# FASCICULE DE BREVET EUROPEEN

(11) **EP 4 271 309 B1**
(45) Date de publication et mention de la délivrance du brevet: **16.10.2024**
(21) Numéro de dépôt: 21844684.7
(22) Date de dépôt: 22.12.2021
(51) Int. Cl.: A61B 34/30, A61B 18/00

(54) **MODULE D'ENTRAINEMENT D'INSTRUMENT MEDICAL SOUPLE ALLONGE DE ROBOT CATHETER**
ANTRIEBSMODUL EINER LÄNGLICHEN FLEXIBLEN MEDIZINISCHEN VORRICHTUNG EINES KATHETERROBOTERS
DRIVE MODULE OF ELONGATE FLEXIBLE MEDICAL DEVICE OF CATHETER ROBOT

(30) Priorité: 29.12.2020 FR 2014222
(43) Date de publication de la demande: 08.11.2023
(73) Titulaire: Robocath, 76000 Rouen (FR)
(72) Inventeur: BOULANGÉ, Marc, 76740 La Gaillarde (FR); HOEFLER, Benoît, 76000 Rouen (FR)
(74) Mandataire: Plasseraud IP
(86) Numéro de dépôt international: PCT/EP2021/087255
(87) Numéro de publication internationale: WO 2022/144267

(56) Documents cités:
- EP-A1- 3 593 851
- WO-A1-2018/176457
- CN-A- 110 236 680
- FR-A1- 3 022 147

## Description

### DOMAINE DE L'INVENTION

L'invention concerne le domaine technique des modules d'entraînement d'instrument médical souple allongé de robot cathéter.

### ARRIERE PLAN TECHNOLOGIQUE DE L'INVENTION

Selon un art antérieur, par exemple décrit dans le brevet FR3037269A1 publié le 16/12/2016, il est connu un module d'entraînement d'instrument médical souple allongé de robot cathéter qui est précis.

Ce module d'entraînement d'instrument médical souple allongé de robot cathéter comprend une chaîne de transmission de mouvement comprenant un socle d'un organe d'entraînement d'un élément mobile, trois actionneurs pilotant le socle de l'organe d'entraînement respectivement selon trois directions de translation distinctes entre elles, par l'intermédiaire de trois interfaces respectives avec le socle de l'organe d'entraînement. Les trois interfaces sont sensiblement planes, orthogonales les unes aux autres, et imbriquées les unes dans les autres.

Mais tout en étant d'un encombrement et d'un poids relativement réduits, ce module d'entraînement d'instrument médical souple allongé de robot cathéter est encore trop volumineux pour certaines applications.

### OBJETS DE L'INVENTION

Le but de la présente invention est de fournir un module d'entraînement d'instrument médical souple allongé de robot cathéter palliant au moins partiellement les inconvénients précités.

Plus particulièrement, l'invention vise à fournir un module d'entraînement d'instrument médical souple allongé de robot cathéter qui soit, d'une part, bien miniaturisé, et d'autre part, bien précis dans les déplacements des porte-touches portant les touches qui viennent saisir et déplacer le ou les instruments médicaux souples allongés insérés dans le robot cathéter.

Pour obtenir un module d'entraînement d'instrument médical souple allongé de robot cathéter présentant ce bon compromis, entre d'une part miniaturisation et d'autre part précision, le module d'entraînement proposé par l'invention :
➢ va, d'une part, présenter une architecture générale comprenant au moins deux dispositifs d'entraînement, un par porte-touche,
   ∘ chaque dispositif d'entraînement comprenant trois blocs d'entraînement, un par direction de translation de chaque porte-touche,
➢ et va, d'autre part, dans cette architecture générale, combiner ensemble plusieurs caractéristiques structurelles particulières qui sont :
   ∘ le caractère autoporteur de chaque dispositif d'entraînement,
   ∘ l'indépendance entre les blocs d'entraînement d'un même dispositif d'entraînement,
   ∘ une structure particulière de blocage des rotations parasites, pour au moins deux blocs d'entraînement par dispositif d'entraînement, intégrant une cinématique de parallélogramme déformable.

A cette fin, la présente invention propose un module d'entraînement d'instrument médical souple allongé de robot cathéter, comprenant : au moins deux dispositifs d'entraînement portant respectivement deux porte-touches situés en vis-à-vis et respectivement destinés à recevoir deux touches qui, en se rapprochant l'une de l'autre, vont venir enserrer l'instrument médical souple allongé pour ensuite l'entraîner en translation et/ou en rotation, chaque dispositif d'entraînement étant autoporteur et comprenant trois blocs d'entraînement qui sont indépendants les uns des autres, entraînant en translation le porte-touche respectivement dans les trois directions orthogonales de l'espace, qui comprennent chacun leur moteur d'entraînement, et au moins deux blocs d'entraînement présentent une structure de blocage des mouvements de rotation du porte-touche : un dispositif d'entraînement autoporteur étant un dispositif d'entraînement dont la structure elle-même assure le maintien en position du porte-touche sans la présence d'un élément de support additionnel, un bloc d'entraînement indépendant des autres blocs d'entraînement étant un bloc d'entraînement qui ne supporte que son propre poids sans supporter aussi le poids d'un autre bloc d'entraînement, une structure de blocage comprenant : une traverse d'entraînement, entraînée par le moteur d'entraînement, une traverse de porte-touche, rigidement reliée au porte-touche, entraînée par la traverse d'entraînement, deux bielles respectivement reliées : d'une part, par une de leurs extrémités, à deux points pivotants de la traverse d'entraînement éloignés l'un de l'autre, d'autre part, par l'autre de leurs extrémités, à deux points pivotants de la traverse de porte-touche éloignés l'un de l'autre, la traverse d'entraînement, la traverse de porte-touche, et les deux bielles, formant ensemble un parallélogramme déformable au niveau de ses quatre sommets qui sont lesdits quatre points pivotants.

Selon des modes de réalisation de l'invention, le module d'entraînement proposé, en plus d'être miniaturisé, réduit le nombre de composants en mouvement, afin de limiter l'inertie et d'améliorer la réactivité du robot cathéter.

Selon des modes de réalisation de l'invention, le dispositif d'entraînement du porte-touche autoporteur, supporte tout seul le porte-touche, et assure tout seul le maintien en position du porte-touche. Le porte-touche garde sa position lorsque le dispositif d'entraînement est éteint. Le porte-touche reste immobile, il n'est pas branlant. Ainsi, le dispositif d'entraînement ne nécessite pas d'élément de support additionnel qui serait destiné à supporter le porte-touche.

Selon des modes de réalisation de l'invention, les blocs cinématiques indépendants ne sont pas montés les uns sur les autres ; en effet, chaque bloc cinématique supporte uniquement son propre poids. Chaque dispositif d'entraînement d'un porte-touche comprend trois blocs cinématiques, un bloc cinématique pour chaque axe des trois mouvements en translation que peut réaliser le porte-touche. Le porte-touche va pouvoir réaliser des mouvements de pure translation selon chacun des trois axes de l'espace. Chaque bloc cinématique a pour fonction de déplacer le porte-touche selon l'un de ces trois axes.

Selon des modes de réalisation de l'invention, afin de bloquer les mouvements de rotation du porte-touche, au moins deux blocs cinématiques forment au moins deux parallélogrammes (et de préférence pas plus que deux parallélogrammes) qui vont contraindre la position du porte-touche et le forcer à rester parallèle au porte-touche situé en vis-à-vis. Il n'est pas utile de former un troisième parallélogramme au niveau du troisième bloc cinématique, car toutes les rotations du porte-touche étant déjà bloquées par les deux premiers parallélogrammes, la structure globale du dispositif d'entraînement peut être simplifiée en ne mettant qu'une seule bielle pour ce troisième bloc cinématique, en évitant la formation d'un tel troisième parallélogramme.

Selon des modes de réalisation de l'invention, chaque bloc cinématique comprend un moteur qui entraine en rotation une manivelle. Une (en l'absence de parallélogramme) ou deux bielles (en présence de parallélogramme) sont fixées à la manivelle via une double liaison pivot. La ou les bielles sont également fixées par une double liaison pivot au porte-touche. Deux des trois blocs cinématiques forment chacun un parallélogramme avec deux bielles, le troisième bloc cinématique ne comprenant qu'une seule bielle. L'utilisation du système bielle et manivelle permet une structure simplifiée. La simplicité du système bielle et manivelle provient notamment du fait que la reprise d'effort peut être facilement assurée par une liaison avec le bâti, en évitant une reprise d'effort qui serait directement réalisée au niveau de l'arbre du moteur lui-même. Le système bielle et manivelle simplifie la structure mécanique, mais demande toutefois une gestion des moteurs qui est un peu plus complexe au niveau logiciel. Cependant, cette complexité du logiciel de gestion des moteurs n'impacte ni le poids, ni l'encombrement du dispositif d'entraînement lequel peut donc être encore plus miniaturisé.

Selon une variante possible des modes de réalisation de l'invention, un ou plusieurs blocs cinématiques peuvent ne pas comprendre de système bielle et manivelle, mais être formé(s) par une plateforme reliée d'une part à un actionneur linéaire, par exemple un moteur à vis sans fin, et d'autre part aux bielles. Cette variante est possible, mais reste toutefois plus complexe mécaniquement, et ne permet pas une miniaturisation aussi poussée que le principal mode de réalisation basé sur l'utilisation d'un système bielle et manivelle.

Pour résoudre la problématique de simplification de la structure du robot cathéter, sans résoudre simultanément la problématique de précision par la gestion des rotations parasites du porte-touches, un autre objet de l'invention, dans un mode de réalisation un peu dégradé, peut concerner un module d'entraînement d'instrument médical souple allongé de robot cathéter, comprenant : au moins deux dispositifs d'entraînement portant respectivement deux porte-touches situés en vis-à-vis et respectivement destinés à recevoir deux touches qui, en se rapprochant l'une de l'autre, vont venir enserrer l'instrument médical souple allongé pour ensuite l'entraîner en translation et/ou en rotation, chaque dispositif d'entraînement étant autoporteur et comprenant trois blocs d'entraînement qui sont indépendants les uns des autres, entraînant en translation le porte-touche respectivement dans les trois directions orthogonales de l'espace, qui comprennent chacun leur moteur d'entraînement : un dispositif d'entraînement autoporteur étant un dispositif d'entraînement dont la structure elle-même assure le maintien en position du porte-touche sans la présence d'un élément de support additionnel, un bloc d'entraînement indépendant des autres blocs d'entraînement étant un bloc d'entraînement qui ne supporte que son propre poids sans supporter aussi le poids d'un autre bloc d'entraînement, et dont au moins deux blocs d'entraînement présentent une structure qui comprend : une traverse d'entraînement, entraînée par le moteur d'entraînement, présentant un axe longitudinal, une traverse de porte-touche, rigidement reliée au porte-touche, entraînée par la traverse d'entraînement, une manivelle entraînée en rotation autour de son axe par le moteur d'entraînement, ledit axe de rotation de la manivelle étant et restant parallèle audit axe longitudinal de la traverse d'entraînement, l'écart inter-axe, c'est-à-dire l'écart entre ledit axe de rotation de la manivelle et ledit axe longitudinal de la traverse d'entraînement, étant et restant constant, de manière à ce que la rotation de la manivelle entraîne une rotation dudit axe longitudinal de la traverse d'entraînement.

Suivant des modes de réalisation préférés, l'invention comprend une ou plusieurs des caractéristiques suivantes qui peuvent être utilisées séparément ou en combinaison partielle entre elles ou en combinaison totale entre elles, l'un ou l'autre des objets de l'invention précités.

De préférence, les deux bielles sont parallèles entre elles.

Ainsi, la forme des bielles permettant de réaliser la cinématique en parallélogramme déformable est la plus simple. Mais d'autres formes de bielles pourraient être envisagées, par exemple des bielles courbées ou des bielles en lignes brisées avec plusieurs segments, même si ces autres formes de bielles seraient moins pratiques.

De préférence, pour au moins un bloc d'entraînement présentant une structure de blocage : la structure de blocage du bloc d'entraînement comprend aussi une manivelle entraînée en rotation autour de son axe par ledit moteur d'entraînement, la traverse d'entraînement présentant un axe longitudinal s'étendant entre ses deux points pivotants, ledit axe de rotation de la manivelle étant et restant parallèle audit axe longitudinal de la traverse d'entraînement, l'écart inter-axe, c'est-à-dire l'écart entre ledit axe de rotation de la manivelle et ledit axe longitudinal de la traverse d'entraînement, étant et restant constant, de manière à ce que la rotation de la manivelle entraîne une rotation dudit axe longitudinal de la traverse d'entraînement.

Ainsi, la structure mécanique du bloc d'entraînement est encore simplifiée, ce qui améliore encore la miniaturisation du module d'entraînement, au prix toutefois d'une certaine complexification de la gestion logicielle de ce module d'entraînement. Mais, globalement, le bénéfice de la miniaturisation l'emporte, et de beaucoup, sur l'inconvénient de la complexification de gestion logicielle.

De préférence, pour au moins deux blocs d'entraînement présentant une structure de blocage, pour chacun de ces deux blocs d'entraînement : la structure de blocage du bloc d'entraînement comprend aussi une manivelle entraînée en rotation autour de son axe par ledit moteur d'entraînement, la traverse d'entraînement présentant un axe longitudinal s'étendant entre ses deux points pivotants, ledit axe de rotation de la manivelle étant et restant parallèle audit axe longitudinal de la traverse d'entraînement, l'écart inter-axe, c'est-à-dire l'écart entre ledit axe de rotation de la manivelle et ledit axe longitudinal de la traverse d'entraînement, étant et restant constant, de manière à ce que la rotation de la manivelle entraîne une rotation dudit axe longitudinal de la traverse d'entraînement.

Ainsi, la structure mécanique du bloc d'entraînement est encore simplifiée, ce qui améliore encore la miniaturisation du module d'entraînement, au prix toutefois d'une certaine complexification de la gestion logicielle de ce module d'entraînement. Mais, globalement, le bénéfice de la miniaturisation l'emporte, et de beaucoup, sur l'inconvénient de la complexification de gestion logicielle.

De préférence, seulement deux blocs d'entraînement, par dispositif d'entraînement, présentent une structure de blocage.

Ainsi, chaque structure étant apte à bloquer deux rotations parasites, en n'utilisant que deux structures de blocage, d'une part les trois rotations parasites sont bloquées, et d'autre part la structure globale du dispositif d'entraînement reste plus simple avec seulement deux structures de blocage plutôt qu'avec trois structures de blocage.

De préférence, pour le troisième bloc d'entraînement ne présentant pas une structure de blocage, la structure dudit bloc d'entraînement comprend : une traverse d'entraînement, entraînée par le moteur d'entraînement, une traverse de porte-touche, rigidement reliée au porte-touche, entraînée par la traverse d'entraînement, une seule bielle respectivement reliée : d'une part, par une de son extrémité, à un point pivotant de la traverse d'entraînement, d'autre part, par son autre extrémité, à un point pivotant de la traverse de porte-touche.

Ainsi, les trois rotations parasites étant déjà bloquées par les deux structures de blocage de deux des trois blocs d'entraînement de chaque dispositif d'entraînement, la structure du troisième bloc d'entraînement de ce dispositif d'entraînement peut être simplifiée, en n'intégrant quant à elle pas de structure de blocage, améliorant de ce fait encore la miniaturisation du module d'entraînement.

De préférence, pour ledit troisième bloc d'entraînement ne présentant pas une structure de blocage : la structure dudit bloc d'entraînement comprend aussi une manivelle entraînée en rotation autour de son axe par ledit moteur d'entraînement, la traverse d'entraînement présentant un axe longitudinal, ledit axe de rotation de la manivelle étant et restant parallèle audit axe longitudinal de la traverse d'entraînement, l'écart inter-axe, c'est-à-dire l'écart entre ledit axe de rotation de la manivelle et ledit axe longitudinal de la traverse d'entraînement, étant et restant constant, de manière à ce que la rotation de la manivelle entraîne une rotation dudit axe longitudinal de la traverse d'entraînement.

Ainsi, la structure du troisième bloc d'entraînement de ce dispositif d'entraînement peut être encore simplifiée, améliorant encore la miniaturisation du module d'entraînement.

De préférence, pour un bloc d'entraînement, ou plusieurs blocs d'entraînement, ou pour tous les blocs d'entraînement : ledit ou lesdits moteurs d'entraînement sont des moteurs rotatifs entraînant en rotation une manivelle rigidement reliée à ladite traverse d'entraînement.

Ainsi, la structure des blocs d'entraînement est rendue encore plus compacte.

Alternativement, pour un bloc d'entraînement, ou plusieurs blocs d'entraînement, ou pour tous les blocs d'entraînement : ledit ou lesdits moteurs d'entraînement sont des moteurs rotatifs entraînant en rotation une vis sans fin de manière à entraîner en translation ladite traverse d'entraînement.

Toutefois, cette alternative est moins compacte que la précédente.

De préférence, pour chaque dispositif d'entraînement : pour chacun des trois blocs d'entraînement dudit dispositif d'entraînement : le bloc d'entraînement entraîne le porte-touche : selon une composante principale de translation suivant une direction principale, ladite direction principale étant différente pour chacun des trois blocs d'entraînement, et selon une composante parasite de translation suivant une autre direction, ladite autre direction étant différente pour chacun des trois blocs d'entraînement, chacun des trois blocs d'entraînement dudit dispositif d'entraînement intègre dans sa composante principale de translation suivant sa direction principale une compensation de la composante parasite de translation d'un des autres blocs d'entraînement suivant cette direction principale.

Ainsi, les actions des trois blocs d'entraînement dudit dispositif d'entraînement s'équilibrent entre elles de manière à ce que le porte-touche reçoive globalement, dans chacune des trois directions de l'espace, la commande de translation requise pour le déplacement voulu dans cette direction, comme résultante dans cette direction de l'ensemble des commandes reçues de la part des trois blocs d'entraînement.

De préférence, lesdits points pivotants sont des articulations pouvant pivoter selon deux degrés de liberté.

D'autres caractéristiques et avantages de l'invention apparaîtront à la lecture de la description qui suit d'un mode de réalisation préféré de l'invention, donnée à titre d'exemple et en référence aux dessins annexés.

### BREVE DESCRIPTION DES DESSINS

**[****Fig. 1****]** La figure 1 représente schématiquement, en perspective, un exemple d'un module d'entraînement d'un instrument médical souple allongé qui comprend deux dispositifs d'entraînement de porte-touche identiques situés l'un en face de l'autre, selon un mode de réalisation de l'invention.
**[****Fig. 2****]** La figure 2 représente schématiquement, en perspective opposée à la perspective de la figure 1, le même exemple de module d'entraînement d'un instrument médical souple allongé qui comprend deux dispositifs d'entraînement de porte-touche identiques situés l'un en face de l'autre, selon un mode de réalisation de l'invention.
**[****Fig. 3****]** La figure 3 représente schématiquement un exemple d'un dispositif d'entraînement d'un module d'entraînement selon les figures 1 et 2, avec représentation des moteurs des différents blocs cinématiques de ce dispositif d'entraînement.
**[****Fig. 4****]** La figure 4 représente schématiquement un exemple d'un dispositif d'entraînement d'un module d'entraînement selon les figures 1 et 2, sans représentation des moteurs des différents blocs cinématiques de ce dispositif d'entraînement.
**[****Fig. 5****]** La figure 5 représente schématiquement un exemple un exemple d'un dispositif d'entraînement d'un module d'entraînement, avec représentation des moteurs des différents blocs cinématiques de ce dispositif d'entraînement, d'une variante possible de la figure 3, variante dans laquelle un bloc cinématique comprend une plateforme déplacée par un actionneur linéaire à la place d'un système bielle et manivelle.
**[****Fig. 6****]** La figure 6 représente schématiquement un exemple de mise en évidence d'un premier parallélogramme déformable de la structure de blocage d'un premier bloc cinématique du dispositif d'entraînement selon la figure 3.
**[****Fig. 7****]** La figure 7 représente schématiquement un exemple de mise en évidence d'un deuxième parallélogramme déformable de la structure de blocage d'un deuxième bloc cinématique du dispositif d'entraînement selon les figures 1 et 2.

### DESCRIPTION DETAILLEE DES MODES DE REALISATION DE L'INVENTION

Dans la description des figures qui suit, l'instrument médical souple allongé peut être par exemple un cathéter guide ou bien un cathéter, par exemple de type ballon ou stent, ou bien un guide de cathéter.

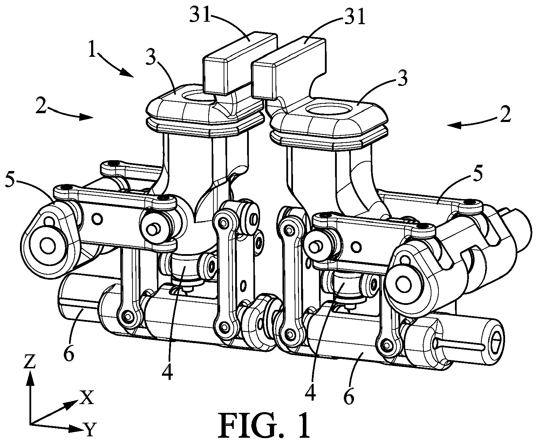

La figure 1 représente schématiquement, en perspective, un exemple d'un module d'entraînement d'un instrument médical souple allongé qui comprend deux dispositifs d'entraînement de porte-touche identiques situés l'un en face de l'autre, selon un mode de réalisation de l'invention.

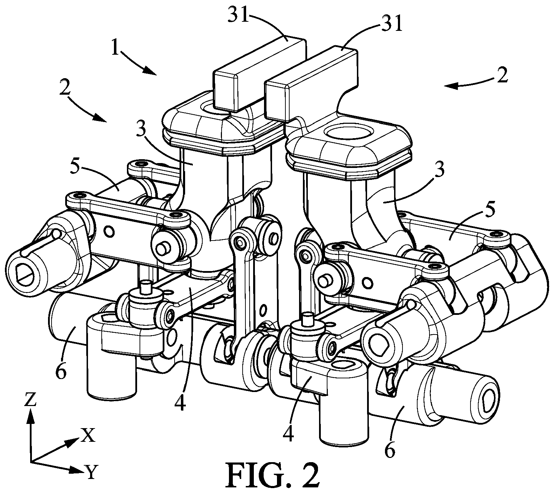

La figure 2 représente schématiquement, en perspective opposée à la perspective de la figure 1, le même exemple de module d'entraînement d'un instrument médical souple allongé qui comprend deux dispositifs d'entraînement de porte-touche identiques situés l'un en face de l'autre, selon un mode de réalisation de l'invention.

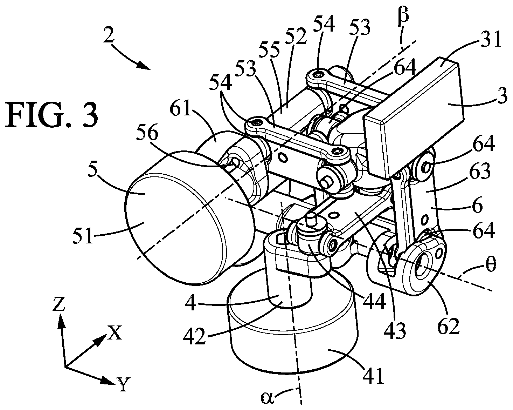

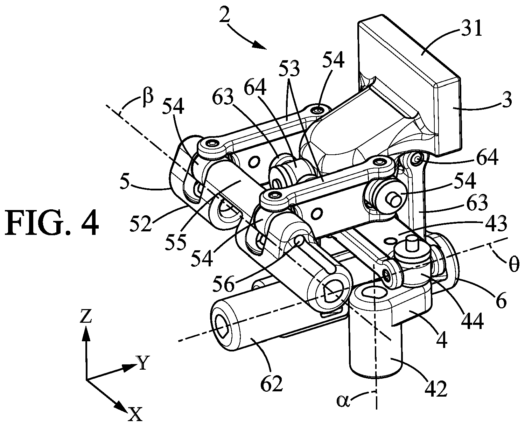

Le module d'entraînement 1 d'un instrument médical souple allongé comprend au moins une paire de dispositifs d'entraînement 2 de porte-touche qui sont identiques entre eux et qui sont situés l'un en face de l'autre. Ces dispositifs d'entraînement 2 de chaque paire sont séparés l'un de l'autre par un espace destiné à recevoir l'instrument médical souple allongé qui sera manipulé par le module d'entraînement 1. Le module d'entraînement 1 peut réaliser une translation et/ou une rotation de l'instrument médical souple allongé. Le module d'entraînement 1 est piloté par une unité de commande 7 qui sera décrite plus en détail ultérieurement, en liaison avec le fonctionnement du module d'entraînement 1 décrit au niveau des figures 3 et 4.

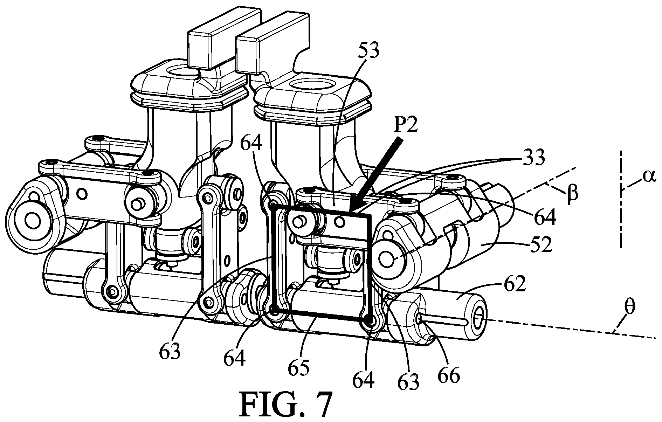

Chaque dispositif d'entraînement 2 comprend un porte-touche 3 qui est un organe qui forme un doigt manipulateur du module d'entraînement 1. Le porte-touche 3 de chaque dispositif d'entraînement 2 est destiné à manipuler l'instrument médical souple allongé en coopérant avec le porte-touche 3 de l'autre dispositif d'entraînement 2 de la paire. La manipulation de l'instrument médical souple allongé par la paire de porte-touches 3, afin de donner un mouvement de translation, de rotation, ou bien un mouvement combiné de translation et de rotation à l'instrument médical souple, est décrit dans la demande de brevet WO2015189531A2 (voir notamment les figures 7a-7e et 8a-8e), publiée le 17/12/2015.

Afin d'assurer la stérilité de l'instrument médical manipulé par les porte-touches 3, une touche stérilisée à usage unique (non représentée sur les figures) est installée sur chaque porte-touche 3 et réalise l'interface entre le porte-touche 3 et l'instrument médical souple allongé, cette touche étant remplacée à chaque nouvelle utilisation du module d'entraînement 1 par une nouvelle touche stérilisée. La touche est installée sur une portion de manipulation 31 du porte-touche 3 qui fait saillie du module d'entraînement 1.

Afin d'assurer la manipulation de l'instrument médical souple allongé, le porte-touche 3 de chaque dispositif d'entraînement 2 peut être entrainé en translation, selon chacun des trois axes orthogonaux x, y et z de l'espace. Afin d'assurer la bonne manipulation de l'instrument médical souple allongé, le porte-touche 3 ne devrait pas subir de mouvement de rotation, afin que les portions de manipulation 31 des porte-touches 3 restent bien parallèles entre elles, ceci afin de bien serrer l'instrument médical souple allongé mais sans tout de même trop le serrer.

Chaque dispositif d'entraînement 2 comprend trois blocs cinématiques 4, 5 et 6, qui sont chacun destinés à déplacer le porte-touche 3 selon un des trois axes orthogonaux x, y et z de l'espace. Chaque dispositif d'entraînement 2 comprend donc, un premier bloc cinématique 4 qui déplace le porte-touche 3 selon l'axe x, un deuxième bloc cinématique 5 qui déplace le porte-touche 3 selon l'axe y, et un troisième bloc cinématique 6 qui déplace le porte-touche 3 selon l'axe z. En outre, l'ensemble des trois blocs cinématiques 4, 5 et 6, forme un système autoporteur qui maintient le porte-touche 3 en position à l'arrêt. Un tel système autoporteur permet d'éviter la présence d'un dispositif additionnel de maintien du porte-touche 3, ce qui simplifie le module d'entraînement 1 tout en réduisant également son encombrement et son poids.

La figure 3 représente schématiquement un exemple d'un dispositif d'entraînement d'un module d'entraînement selon les figures 1 et 2, avec représentation des moteurs des différents blocs cinématiques de ce dispositif d'entraînement.

La figure 4 représente schématiquement un exemple d'un dispositif d'entraînement d'un module d'entraînement selon les figures 1 et 2, sans représentation des moteurs des différents blocs cinématiques de ce dispositif d'entraînement.

Le premier bloc cinématique 4 comprend un premier moteur 41 qui entraine en rotation une première manivelle 42 selon un axe de rotation α qui est dirigé selon l'axe z. Une bielle 43 est fixée à la manivelle 42 via une double liaison pivot 44, la fixation entre la bielle 43 et la manivelle 42, au niveau de la double liaison pivot 44, étant déportée de l'axe de rotation α de la manivelle 42 et du premier moteur 41. La bielle 43 est fixée à son autre extrémité au porte-touche 3 via une double liaison pivot 44 identique à celle réalisant la liaison entre la bielle 43 et la manivelle 42. Les doubles liaisons pivot 44 sont des liaisons qui autorisent d'une part une rotation selon l'axe z, et d'autre part une rotation selon un axe perpendiculaire à l'axe z. Une rotation du premier moteur 41 entraine ainsi un déplacement du porte-touche 3 qui est principalement une translation effectuée selon l'axe x.

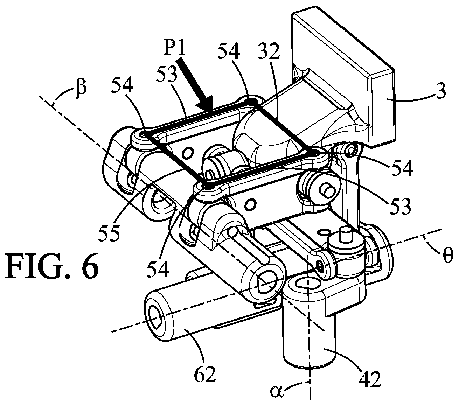

Le deuxième bloc cinématique 5 comprend un deuxième moteur 51 qui entraine en rotation une deuxième manivelle 52 selon un axe de rotation β qui est dirigé selon l'axe x. Deux bielles 53 sont fixées à la manivelle 52, chacune des bielles 53 via une double liaison pivot 54, la fixation, au niveau de cette double liaison pivot 54, entre chacune des bielles 53 et la manivelle 52 étant déportée de l'axe de rotation β de la manivelle 52 et du deuxième moteur 51. Les bielles 53 sont chacune fixées, à leur autre extrémité, au porte-touche 3 via une double liaison pivot 54 identique à celles réalisant la liaison avec la manivelle 52. Les bielles 53 sont parallèles et sont situées de part et d'autre du porte-touche 3 de sorte à l'entourer. Les bielles 53, l'axe 56 ou la traverse 55 de la manivelle 52 et le porte-touche 3 forment ensemble un parallélogramme, qui sera décrit avec plus de détail sur la figure 6, les deux bielles 53 formant deux côtés opposés et la manivelle 52 et le porte-touche 3 formant les deux autres côtés opposés. La traverse 55 de la manivelle 52 est une matérialisation de cette partie 55 de la manivelle 52 qui est située entre les deux doubles liaisons pivot 54.

Les doubles liaisons pivot 54 sont des liaisons qui autorisent d'une part une rotation selon l'axe x, et d'autre part une rotation selon un axe perpendiculaire à l'axe x. Une rotation du deuxième moteur 51 entraine ainsi un déplacement du porte-touche 3 qui est principalement une translation effectuée selon l'axe y.

Le troisième bloc cinématique 6 comprend un troisième moteur 61 qui entraine en rotation une troisième manivelle 62 selon un axe de rotation θ qui est dirigé selon l'axe y. Deux bielles 63 sont fixées à la manivelle 62, chacune des bielles 63 via une double liaison pivot 64, la fixation, au niveau de la double liaison 64, entre chacune des bielles 63 et la manivelle 62 étant déportée de l'axe de rotation θ de la manivelle 62 et du deuxième moteur 61. Les bielles 63 sont chacune fixées à leur autre extrémité au porte-touche 3 via une double liaison pivot 64 identique à celles réalisant la liaison avec la manivelle 62. Les bielles 63 sont parallèles et sont situées de part et d'autre du porte-touche 3 de sorte à l'entourer. Les bielles 63, la manivelle 62 et le porte-touche 3 forment ensemble un parallélogramme, qui sera décrit avec plus de détail sur la figure 7, les deux bielles 63 formant deux côtés opposés et la manivelle 62 et le porte-touche 3 formant les deux autres côtés opposés. Les doubles liaisons pivot 64 sont des liaisons qui autorisent d'une part une rotation selon l'axe y, et d'autre part une rotation selon un axe perpendiculaire à l'axe y. Une rotation du deuxième moteur 61 entraine ainsi un déplacement du porte-touche 3 qui est principalement une translation effectuée selon l'axe z.

Dans une réalisation, le premier moteur 41, le deuxième moteur 51 et le troisième moteur 61, sont des moteurs rotatifs, ce qui permet de simplifier le dispositif d'entraînement 2. Selon une autre variante possible, les manivelles 42, 52 et 62, peuvent être entrainées en rotation par un moteur linéaire dont la liaison avec chacune de ces manivelles 42, 52 et 62, est déportée de l'axe de rotation de cette manivelle de la même manière que les bielles sur les figures 3 et 4.

La structure bielle et manivelle des blocs cinématiques 4, 5 et 6, permet en outre de simplifier la reprise des efforts sans solliciter les moteurs. Une solution simple est de disposer, pour chaque bielle, une liaison pivot qui relie cette bielle au bâti, la liaison pivot étant située à l'extrémité de la manivelle qui est opposée à l'extrémité de la bielle qui est reliée au moteur.

Dans le mode de réalisation représenté sur les figures 1 à 4, l'actionnement d'un bloc cinématique ne déplace pas le porte-touche 3 en translation seulement selon un unique axe, le mouvement du porte-touche 3 étant en effet une translation qui comprend une composante principale selon un axe et une composante secondaire selon un autre axe. Cette composante secondaire, nettement moins importante que la composante principale, est une composante parasite qui va être compensée. Par exemple, l'actionnement du deuxième bloc cinématique 5 avec la rotation du moteur 51 entraine un mouvement de translation du porte-touche 3 avec une composante principale d'axe y et une composante secondaire d'axe z.

Afin de compenser cette composante secondaire, le module d'entraînement 1 comprend une unité de commande 7 qui commande le fonctionnement des blocs cinématiques 4, 5 et 6, l'unité de commande 7 activant les blocs cinématiques 4, 5 et 6, afin que le mouvement du porte-touche 3 corresponde à la commande de l'utilisateur. Par exemple, si l'utilisateur commande un pur mouvement de rotation du cathéter, c'est-à-dire un pur mouvement de translation selon l'axe z du porte-touche 3, l'unité de commande 7 commande simultanément l'activation des trois blocs cinématiques 4, 5 et 6. Le troisième bloc cinématique 6 est activé pour donner au porte-touche un mouvement de translation avec une composante principale d'axe z et une composante secondaire d'axe x. Afin de compenser la composante secondaire d'axe x, le premier bloc cinématique 4 est activé afin de maintenir fixe le porte-touche 3 selon l'axe x, l'activation du premier bloc cinématique 4 créant toutefois une composante secondaire d'axe y. Afin de compenser la composante secondaire d'axe y, le deuxième bloc cinématique 5 est également activé afin de maintenir fixe le porte-touche 3 selon l'axe y, l'activation du deuxième bloc cinématique 5 crée toutefois une composante secondaire d'axe z qui est prise en compte par l'unité de commande 7 pour sa consigne aux blocs cinématiques 4, 5 et 6. Les trois blocs cinématiques 4, 5 et 6, sont activés simultanément d'une part afin de générer toutes les composantes principales voulues par l'utilisateur et d'autre part afin de compenser toutes les composantes secondaires parasites non voulues par l'utilisateur.

Dans le mode de réalisation représenté sur les figures 1 à 4, la structure mécanique du module d'entraînement 1 est simplifiée, ce qui complexifie cependant l'algorithme de gestion de l'activation des blocs cinématiques 4, 5 et 6, en fonction du mouvement demandé par l'utilisateur mis en oeuvre par l'unité de commande 7.

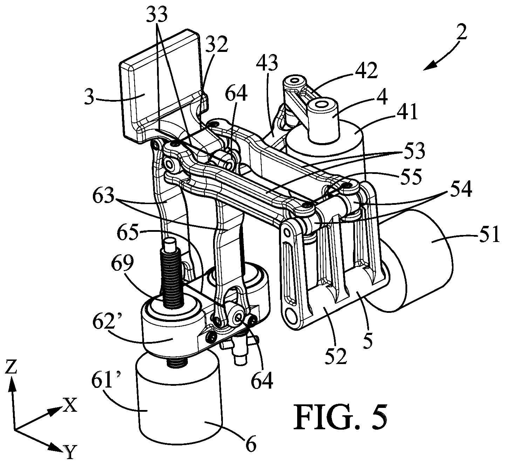

La figure 5 représente schématiquement un exemple un exemple d'un dispositif d'entraînement d'un module d'entraînement, avec représentation des moteurs des différents blocs cinématiques de ce dispositif d'entraînement, d'une variante possible de la figure 3, variante dans laquelle un bloc cinématique comprend une plateforme déplacée par un actionneur linéaire à la place d'un système bielle et manivelle.

Selon une variante possible qui est illustrée sur la figure 5, un ou plusieurs blocs cinématiques 4, 5 et 6, peuvent ne pas être formés par un système bielle et manivelle. Le dispositif d'entraînement 2 présente un troisième bloc cinématique 6 qui a pour fonction de déplacer le porte-touche 3 selon l'axe z et qui comprend une plateforme 62' qui est entraînée en translation selon l'axe z par un actionneur linéaire 61'. La plateforme 62' est bloquée en rotation selon n'importe lequel des trois axes, et est également bloquée pour les translations selon l'axe x et l'axe y. De manière similaire à la manivelle 62 du mode de réalisation des figures 1 à 4, la plateforme 62' est reliée à deux bielles 63 via des doubles liaisons pivot 64. Les bielles 63 sont également reliées au porte-touche 3 via des doubles liaisons pivot 64. Les doubles liaisons pivot 64 sont des liaisons qui autorisent d'une part une rotation selon l'axe y, et d'autre part une rotation selon un axe perpendiculaire à l'axe y.

Sur la figure 5, un premier parallélogramme est formé par les deux bielles 63, la plateforme 62' et le porte-touche 3, en fait plus précisément par les deux bielles 63, la traverse d'entraînement 65 de la plateforme 62' et la traverse 33 du porte-touche 3. Ce premier parallélogramme bloque les mouvements de rotation du porte-touche 3 autour des axes x et z. La traverse 65 de la manivelle 62 est une matérialisation de cette partie 65 de la manivelle 62 qui est située entre les deux doubles liaisons pivot 64. D'une manière similaire au mode de réalisation présenté sur les figures 1 à 4, le deuxième parallélogramme est formé par les bielles 53, la manivelle 52 et le porte-touche 3, en fait plus précisément par les deux bielles 53, la traverse d'entraînement 55 de la manivelle 52 et la traverse 32 du porte-touche 3.

L'actionneur 61' est un moteur à vis sans fin 69 dans la variante illustrée sur la figure 5, d'autres types d'actionneurs linéaires peuvent toutefois être utilisés à sa place.

La variante illustrée sur la figure 5 n'offre pas la même simplicité mécanique que la variante des figures 1 à 4, notamment concernant le positionnement d'une liaison avec la plateforme 62' pour la reprise d'effort sans solliciter l'actionneur 61'.

La figure 6 représente schématiquement un exemple de mise en évidence d'un premier parallélogramme déformable de la structure de blocage d'un premier bloc cinématique du dispositif d'entraînement selon la figure 3.

Les bielles 53, l'axe 56 ou la traverse 55 de la manivelle 52 et la traverse 32 du porte-touche 3 forment ensemble un premier parallélogramme P1, les deux bielles 53 formant deux côtés opposés et l'axe 56 ou la traverse 55 de la manivelle 52 et la traverse 32 du porte-touche 3 formant les deux autres côtés opposés. Les quatre côtés du premier parallélogramme P1 peuvent bouger les uns par rapport aux autres en pivotant autour des doubles liaisons 54, mais toujours en conservant parallèles entre eux les deux bielles 53 d'une part et parallèles entre eux l'axe 56 ou la traverse 55 de la manivelle 52 et la traverse 32 du porte-touche 3. Le premier parallélogramme P1 peut donc se déformer par pivotement de ses quatre côtés autour de ses quatre sommets que sont les quatre double liaisons 54, mais reste toujours un parallélogramme.

Le premier parallélogramme P1 a été choisi en liaison avec l'axe de rotation β, mais il aurait pu être choisi soit en liaison avec l'axe de rotation θ soit avec l'axe de rotation α.

La figure 7 représente schématiquement un exemple de mise en évidence d'un deuxième parallélogramme déformable de la structure de blocage d'un deuxième bloc cinématique du dispositif d'entraînement selon les figures 1 et 2.

Les bielles 63, l'axe 66 ou la traverse 65 de la manivelle 52 et la traverse 33 du porte-touche 3 forment ensemble un deuxième parallélogramme P2, les deux bielles 63 formant deux côtés opposés et l'axe 66 ou la traverse 65 de la manivelle 62 et la traverse 33 du porte-touche 3 formant les deux autres côtés opposés. Les quatre côtés du deuxième parallélogramme P2 peuvent bouger les uns par rapport aux autres en pivotant autour des doubles liaisons 64, mais toujours en conservant parallèles entre eux les deux bielles 63 d'une part et parallèles entre eux l'axe 66 ou la traverse 65 de la manivelle 62 et la traverse 33 du porte-touche 3. Le deuxième parallélogramme P2 peut donc se déformer par pivotement de ses quatre côtés autour de ses quatre sommets que sont les quatre double liaisons 64, mais reste toujours un parallélogramme.

Le deuxième parallélogramme P2 a été choisi en liaison avec l'axe de rotation θ, mais il aurait pu être choisi soit en liaison avec l'axe de rotation β soit avec l'axe de rotation α.

La structure à double parallélogrammes du dispositif d'entraînement 2, les parallélogrammes P1 et P2 respectivement décrits plus en détail aux figures 6 et 7, est une solution structurellement simple qui permet de bloquer tous les mouvements de rotation du porte-touche 3, tout en autorisant tous les mouvements de translation dudit porte-touche 3. En effet, chaque parallélogramme, P1 ou P2, bloque deux axes de rotation du porte-touche 3, les trois axes de rotation étant bloqués en additionnant les deux parallélogrammes.

Le premier parallélogramme P1, qui est formé par les bielles 53, la manivelle 52 et la traverse 32 du porte-touche 3 bloque les rotations dudit porte-touche 3 selon les axes y et z. La traverse 32 du porte-touche 3 n'est qu'une matérialisation d'une partie du porte-touche 3 reliant les deux double liaisons pivot 54. En effet, le porte-touche 3 est maintenu parallèle à la manivelle 52, et comme la manivelle 52 est uniquement mobile en rotation autour de l'axe β (et donc l'axe x), les mouvements de rotation du porte-touche 3 autour des axes y et z sont bloqués.

Le second parallélogramme P2, qui est formé par les bielles 63, la manivelle 63 et la traverse 33 du porte-touche 3 bloque les rotations dudit porte-touche 3 selon les axes x et z. La traverse 33 du porte-touche 3 n'est qu'une matérialisation d'une partie du porte-touche 3 reliant les deux double liaisons pivot 64. En effet, le porte-touche 3 est maintenu parallèle à la manivelle 62, et comme la manivelle 62 est uniquement mobile en rotation autour de l'axe θ (et donc l'axe y), les mouvements de rotation du porte-touche 3 autour des axes x et z sont bloqués. Pour des raisons de visibilité, sur la figure 7, le second parallélogramme P2 est montré devant la bielle 53 du deuxième bloc cinématique tandis qu'en réalité, il se trouve derrière cette bielle 53, puisqu'en effet, les quatre sommets du second parallélogramme P2 sont les quatre double liaisons 64.

La structure ne comprend pas de troisième parallélogramme, car l'utilisation d'une unique bielle 43 au niveau de l'axe de rotation α permet de simplifier la structure, les trois axes de rotation x, y et z, étant déjà bloqués par les deux parallélogrammes P1 et P2.

Bien entendu, la présente invention n'est pas limitée aux exemples et au mode de réalisation décrits et représentés, mais elle est susceptible de nombreuses variantes accessibles à l'homme de l'art dans la limite des revendications jointes.

## Revendications

1. Module d'entraînement d'instrument médical souple allongé de robot cathéter, comprenant :
> au moins deux dispositifs d'entraînement (2) portant respectivement deux porte-touches (3) situés en vis-à-vis et respectivement destinés à recevoir deux touches qui, en se rapprochant l'une de l'autre, vont venir enserrer l'instrument médical souple allongé pour ensuite l'entraîner en translation et/ou en rotation,
> chaque dispositif d'entraînement (2) étant autoporteur et comprenant trois blocs d'entraînement (4, 5, 6) qui sont indépendants les uns des autres, entraînant en translation le porte-touche (3) respectivement dans les trois directions orthogonales de l'espace (x, y, z), qui comprennent chacun leur moteur d'entraînement (41, 51, 61), et au moins deux blocs d'entraînement (5, 6) présentent une structure de blocage des mouvements de rotation du porte-touche (3) :
∘ un dispositif d'entraînement autoporteur étant un dispositif d'entraînement dont la structure elle-même assure le maintien en position du porte-touche sans la présence d'un élément de support additionnel,
∘ un bloc d'entraînement indépendant des autres blocs d'entraînement étant un bloc d'entraînement qui ne supporte que son propre poids sans supporter aussi le poids d'un autre bloc d'entraînement,
∘ une structure de blocage comprenant :
▪ une traverse d'entraînement (55, 65), entraînée par le moteur d'entraînement (51, 61),
▪ une traverse (32, 33) de porte-touche (3), rigidement reliée au porte-touche (3), entraînée par la traverse d'entraînement (55, 65),
▪ deux bielles (53, 63) respectivement reliées :
• d'une part, par une de leurs extrémités, à deux points pivotants (54, 64) de la traverse d'entraînement (55, 65) éloignés l'un de l'autre,
• d'autre part, par l'autre de leurs extrémités, à deux points pivotants (54, 64) de la traverse (32, 33) de porte-touche (3) éloignés l'un de l'autre,
▪ la traverse d'entraînement (55, 65), la traverse (32, 33) de porte-touche (3), et les deux bielles (53, 63), formant ensemble un parallélogramme (P1, P2) déformable au niveau de ses quatre sommets qui sont lesdits quatre points pivotants (54, 64).

2. Module d'entraînement selon la revendication 1, **caractérisé en ce que** les deux bielles (53, 63) sont parallèles entre elles.

3. Module d'entraînement selon l'une quelconque des revendications précédentes, **caractérisé en ce que**, pour au moins un bloc d'entraînement (5, 6) présentant une structure de blocage :
> la structure de blocage du bloc d'entraînement (5, 6) comprend aussi une manivelle (52, 62) entraînée en rotation autour de son axe par ledit moteur d'entraînement (51, 61),
> la traverse d'entraînement (55, 65) présentant un axe longitudinal s'étendant entre ses deux points pivotants (54, 64),
> ledit axe de rotation de la manivelle (52, 62) étant et restant parallèle audit axe longitudinal de la traverse d'entraînement (55, 65),
> l'écart inter-axe, c'est-à-dire l'écart entre ledit axe de rotation de la manivelle (52, 62) et ledit axe longitudinal de la traverse d'entraînement (55, 65), étant et restant constant, de manière à ce que la rotation de la manivelle (52, 62) entraîne une rotation dudit axe longitudinal de la traverse d'entraînement (55, 65).

4. Module d'entraînement selon la revendication 3, **caractérisé en ce que**, pour au moins deux blocs d'entraînement (5, 6) présentant une structure de blocage, pour chacun de ces deux blocs d'entraînement (5, 6) :
> la structure de blocage du bloc d'entraînement (5, 6) comprend aussi une manivelle (52, 62) entraînée en rotation autour de son axe par ledit moteur d'entraînement (51, 61),
> la traverse d'entraînement (55, 65) présentant un axe longitudinal s'étendant entre ses deux points pivotants (54, 64),
> ledit axe de rotation de la manivelle (52, 62) étant et restant parallèle audit axe longitudinal de la traverse d'entraînement (55, 65),
> l'écart inter-axe, c'est-à-dire l'écart entre ledit axe de rotation de la manivelle (52, 62) et ledit axe longitudinal de la traverse d'entraînement (55, 65), étant et restant constant, de manière à ce que la rotation de la manivelle (52, 62) entraîne une rotation dudit axe longitudinal de la traverse d'entraînement (55, 65).

5. Module d'entraînement selon la revendication 4, **caractérisé en ce que**, seulement deux blocs d'entraînement (5, 6), par dispositif d'entraînement (2), présentent une structure de blocage.

6. Module d'entraînement selon la revendication 5, **caractérisé en ce que**, pour le troisième bloc d'entraînement (4) ne présentant pas une structure de blocage, la structure dudit bloc d'entraînement (4) comprend :
➢ une traverse d'entraînement, entraînée par le moteur d'entraînement (41),
➢ une traverse de porte-touche (3), rigidement reliée au porte-touche (3), entraînée par la traverse d'entraînement,
➢ une seule bielle (43) respectivement reliée :
∘ d'une part, par une de son extrémité, à un point pivotant (44) de la traverse d'entraînement,
∘ d'autre part, par son autre extrémité, à un point pivotant (44) de la traverse de porte-touche (3).

7. Module d'entraînement selon la revendication 6, **caractérisé en ce que**, pour ledit troisième bloc d'entraînement (4) ne présentant pas une structure de blocage :
> la structure dudit bloc d'entraînement (4) comprend aussi une manivelle (42) entraînée en rotation autour de son axe par ledit moteur d'entraînement (41),
> la traverse d'entraînement présentant un axe longitudinal,
> ledit axe de rotation de la manivelle (42) étant et restant parallèle audit axe longitudinal de la traverse d'entraînement,
> l'écart inter-axe, c'est-à-dire l'écart entre ledit axe de rotation de la manivelle (42) et ledit axe longitudinal de la traverse d'entraînement, étant et restant constant, de manière à ce que la rotation de la manivelle (42) entraîne une rotation dudit axe longitudinal de la traverse d'entraînement.

8. Module d'entraînement selon l'une quelconque des revendications précédentes, **caractérisé en ce que**, pour un bloc d'entraînement (4, 5, 6), ou plusieurs blocs d'entraînement (4, 5, 6), ou pour tous les blocs d'entraînement (4, 5, 6), ledit ou lesdits moteurs d'entraînement (41, 51, 61) sont des moteurs rotatifs entraînant en rotation une manivelle (42, 52, 62) rigidement reliée à ladite traverse d'entraînement (55, 65).

9. Module d'entraînement selon l'une quelconque des revendications précédentes, **caractérisé en ce que**, pour un bloc d'entraînement (4, 5, 6), ou plusieurs blocs d'entraînement (4, 5, 6), ou pour tous les blocs d'entraînement (4, 5, 6), ledit ou lesdits moteurs d'entraînement (41, 51, 61) sont des moteurs rotatifs entraînant en rotation une vis sans fin (69) de manière à entraîner en translation ladite traverse d'entraînement (55, 65).

10. Module d'entraînement selon l'une quelconque des revendications précédentes, **caractérisé en ce que**, pour chaque dispositif d'entraînement (2) :
➢ pour chacun des trois blocs d'entraînement (4, 5, 6) dudit dispositif d'entraînement (2) :
∘ le bloc d'entraînement (4, 5, 6) entraîne le porte-touche (3) :
▪ selon une composante principale de translation suivant une direction principale, ladite direction principale étant différente pour chacun des trois blocs d'entraînement (4, 5, 6),
▪ et selon une composante parasite de translation suivant une autre direction, ladite autre direction étant différente pour chacun des trois blocs d'entraînement (4, 5, 6),
> chacun des trois blocs d'entraînement (4, 5, 6) dudit dispositif d'entraînement (2) intègre dans sa composante principale de translation suivant sa direction principale une compensation de la composante parasite de translation d'un des autres blocs d'entraînement (4, 5, 6) suivant cette direction principale.

11. Module d'entraînement selon l'une quelconque des revendications précédentes, **caractérisé en ce que** lesdits points pivotants (54, 64) sont des articulations pouvant pivoter selon deux degrés de liberté.

## Patentansprüche

1. Antriebsmodul für ein langgestrecktes, flexibles medizinisches Instrument eines Katheterroboters, umfassend:
> wenigstens zwei Antriebsvorrichtungen (2), die jeweils zwei gegenüberliegende Tastenhalter (3) tragen, die jeweils dazu bestimmt sind, zwei Tasten aufzunehmen, die, indem sie sich einander annähern, das längliche flexible medizinische Instrument umschließen, um es anschließend in Translation und/oder Rotation anzutreiben,
> wobei jede Antriebsvorrichtung (2) selbsttragend ist und drei Antriebsblöcke (4, 5, 6) umfasst, die voneinander unabhängig sind und den Tastenhalter (3) jeweils in den drei orthogonalen Raumrichtungen (x, y, z) translatorisch antreiben, welche jeweils ihren Antriebsmotor (41, 51, 61) umfassen, wobei wenigstens zwei Antriebsblöcke (5, 6) eine Struktur zur Blockierung der Rotationsbewegungen des Tastenhalters (3) aufweisen:
∘ wobei eine selbsttragende Antriebseinheit eine Antriebseinheit ist, deren Struktur selbst sicherstellt, dass der Tastenhalter ohne das Vorhandensein eines zusätzlichen Stützelementes in Position gehalten wird,
∘ wobei ein von anderen Antriebsblöcken unabhängiger Antriebsblock ein Antriebsblock ist, der nur sein eigenes Gewicht trägt, ohne auch das Gewicht eines anderen Antriebsblocks zu tragen,
∘ wobei eine Blockierstruktur umfasst:
▪ eine Antriebstraverse (55, 65), die von dem Antriebsmotor (51, 61) angetrieben wird,
▪ eine Traverse (32, 33) des Tastenhalters (3), die starr mit dem Tastenhalter (3) verbunden ist und von der Antriebstraverse (55, 65) angetrieben wird,
▪ zwei Pleuelstangen (53, 63), die jeweils verbunden sind:
• einerseits durch eines ihrer Enden mit zwei voneinander entfernten Schwenkpunkten (54, 64) der Antriebstraverse (55, 65),
• andererseits mit dem anderen ihrer Enden an zwei voneinander entfernten Schwenkpunkten (54, 64) der Traverse (32, 33) des Tastenhalters (3),
▪ wobei die Antriebstraverse (55, 65), die Traverse (32, 33) des Tastenhalters (3) und die beiden Pleuelstangen (53, 63) zusammen ein Parallelogramm (P1, P2) bilden, das an seinen vier Eckpunkten, die die vier Schwenkpunkte (54, 64) sind, verformbar ist.

2. Antriebsmodul nach Anspruch 1, **dadurch gekennzeichnet, dass** die beiden Pleuelstangen (53, 63) parallel zueinander sind.

3. Antriebsmodul nach einem der vorhergehenden Ansprüche, **dadurch gekennzeichnet, dass** bei wenigstens einem Antriebsblock (5, 6), der eine Blockierstruktur aufweist:
➢ die Blockierstruktur des Antriebsblocks (5, 6) ferner eine Kurbel (52, 62) umfasst, die durch den Antriebsmotor (51, 61) um ihre Achse zur Rotation angetrieben wird,
➢ die Antriebstraverse (55, 65) eine Längsachse aufweist, die sich zwischen ihren beiden Schwenkpunkten (54, 64) erstreckt,
➢ wobei die Rotationsachse der Kurbel (52, 62) parallel zu der Längsachse der Antriebstraverse (55, 65) ist und bleibt,
➢ wobei der Zwischenachsenabstand, d.h. der Abstand zwischen der Rotationsachse der Kurbel (52, 62) und der Längsachse der Antriebstraverse (55, 65), konstant ist und bleibt, so dass die Rotation der Kurbel (52, 62) eine Rotation der Längsachse der Antriebstraverse (55, 65) bewirkt.

4. Antriebsmodul nach Anspruch 3, **dadurch gekennzeichnet, dass** bei wenigstens zwei Antriebsblöcken (5, 6), die eine Blockierstruktur aufweisen, bei jedem dieser zwei Antriebsblöcke (5, 6):
> die Blockierstruktur des Antriebsblocks (5, 6) ferner eine Kurbel (52, 62) umfasst, die durch den Antriebsmotor (51, 61) um ihre Achse zur Rotation angetrieben wird,
> die Antriebstraverse (55, 65) eine Längsachse aufweist, die sich zwischen ihren beiden Schwenkpunkten (54, 64) erstreckt,
> wobei die Rotationsachse der Kurbel (52, 62) parallel zu der Längsachse der Antriebstraverse (55, 65) ist und bleibt,
➢ wobei der Zwischenachsabstand, d. h. der Abstand zwischen der Rotationsachse der Kurbel (52, 62) und der Längsachse der Antriebstraverse (55, 65), konstant ist und bleibt, so dass die Rotation der Kurbel (52, 62) eine Rotation der Längsachse der Antriebstraverse (55, 65) bewirkt.

5. Antriebsmodul nach Anspruch 4, **dadurch gekennzeichnet, dass** nur zwei Antriebsblöcke (5, 6) pro Antriebsvorrichtung (2) eine Blockierstruktur aufweisen.

6. Antriebsmodul nach Anspruch 5, **dadurch gekennzeichnet, dass** bei dem dritten Antriebsblock (4), der keine Blockierstruktur aufweist, die Struktur des Antriebsblocks (4) umfasst:
➢ eine Antriebstraverse, die von dem Antriebsmotor (41) angetrieben wird,
➢ eine Traverse des Tastenhalters (3), die starr mit dem Tastenhalter (3) verbunden ist und von der Antriebstraverse angetrieben wird,
➢ eine einzelne Pleuelstange (43), die jeweils verbunden ist:
∘ einerseits, durch eines ihrer Enden, mit einem Schwenkpunkt (44) der Antriebstraverse,
∘ andererseits mit ihrem anderen Ende mit einem Schwenkpunkt (44) der Traverse des Tastenhalters (3).

7. Antriebsmodul nach Anspruch 6, **dadurch gekennzeichnet, dass** bei dem dritten Antriebsblock (4), der keine Blockierstruktur aufweist:
➢ die Struktur des Antriebsblocks (4) ferner eine Kurbel (42) umfasst, die von dem Antriebsmotor (41) um ihre Achse zur rotation angetrieben wird,
➢ wobei die Antriebstraverse eine Längsachse aufweist,
➢ wobei die Rotationsachse der Kurbel (42) parallel zur Längsachse der Antriebstraverse ist und bleibt,
➢ wobei der Zwischenachsabstand, d. h. der Abstand zwischen der Rotationsachse der Kurbel (42) und der Längsachse der Antriebstraverse, konstant ist und bleibt, so dass die Rotation der Kurbel (42) eine Rotation der Längsachse der Antriebstraverse bewirkt.

8. Antriebsmodul nach einem der vorhergehenden Ansprüche, **dadurch gekennzeichnet, dass** bei einem Antriebsblock (4, 5, 6) oder mehreren Antriebsblöcken (4, 5, 6) oder bei allen Antriebsblöcke (4, 5, 6) der oder die Antriebsmotoren (41, 51, 61) Drehmotoren sind, die eine Kurbel (42, 52, 62), die starr mit der Antriebstraverse (55, 65) verbunden ist, zur Rotation antreiben.

9. Antriebsmodul nach einem der vorhergehenden Ansprüche, **dadurch gekennzeichnet, dass** bei einem Antriebsblock (4, 5, 6) oder mehreren Antriebsblöcken (4, 5, 6) oder bei allen Antriebsblöcken (4, 5, 6) der oder die Antriebsmotoren (41, 51, 61) Rotationsmotoren sind, die eine Schnecke (69) zur Rotation antreiben, um die Antriebstraverse (55, 65) zur Translation anzutreiben.

10. Antriebsmodul nach einem der vorhergehenden Ansprüche, **dadurch gekennzeichnet, dass** bei jeder Antriebsvorrichtung (2):
➢ bei jedem der drei Antriebsblöcke (4, 5, 6) der Antriebsvorrichtung (2):
∘ der Antriebsblock (4, 5, 6) den Tastenhalter (3) antreibt:
▪ gemäß einer translatorischen Hauptkomponente entlang einer Hauptrichtung, wobei die Hauptrichtung bei jedem der drei Antriebsblöcke (4, 5, 6) unterschiedlich ist,
▪ und gemäß einer parasitären Translationskomponente entlang einer anderen Richtung, wobei die andere Richtung bei jedem der drei Antriebsblöcke (4, 5, 6) unterschiedlich ist,
➢ jeder der drei Antriebsblöcke (4, 5, 6) der Antriebsvorrichtung (2) in seiner Haupttranslationskomponente entlang seiner Hauptrichtung eine Kompensation der parasitären Translationskomponente eines der anderen Antriebsblöcke (4, 5, 6) entlang dieser Hauptrichtung integriert.

11. Antriebsmodul nach einem der vorhergehenden Ansprüche, **dadurch gekennzeichnet, dass** die Schwenkpunkte (54, 64) Gelenke sind, die in zwei Freiheitsgraden schwenkbar sind.

## Claims

1. A drive module for an elongate flexible medical device of a catheter robot, comprising:
> at least two drive devices (2) respectively carrying two pad holders (3) located opposite one another and respectively intended to receive two pads which, as they approach each other, will grip the elongate flexible medical device in order to then drive it in translation and/or in rotation,
> each drive device (2) being self-supporting and comprising three drive units (4, 5, 6) which are independent of each other, driving the pad holder (3) in translation respectively in the three orthogonal spatial directions (x, y, z), which each comprise their drive motor (41, 51, 61), and at least two drive units (5, 6) have a blocking structure preventing rotational movements of the pad holder (3):
∘ a self-supporting drive device being a drive device in which its own structure ensures that the pad holder is held in position without the presence of an additional support element,
∘ a drive unit that is independent of the other drive units being a drive unit which only supports its own weight without also supporting the weight of another drive unit,
∘ a rotation-preventing structure comprising:
▪ a drive crossmember (55, 65), driven by the drive motor (51, 61),
▪ a pad holder (3) crossmember (32, 33), rigidly connected to the pad holder (3) and driven by the drive crossmember (55, 65),
▪ two connecting rods (53, 63) respectively connected:
• by one of their ends to two pivoting points (54, 64) of the drive crossmember (55, 65) which are set apart from each other,
• by the other of their ends to two pivoting points (54, 64) of the pad holder (3) crossmember (32, 33) which are set apart from each other,
▪ the drive crossmember (55, 65), the pad holder (3) crossmember (32, 33), and the two connecting rods (53, 63) together forming a parallelogram (P1, P2) that is deformable at its four vertices which are said four pivoting points (54, 64).

2. The drive module according to claim 1, **characterized in that** the two connecting rods (53, 63) are parallel to each other.

3. The drive module according to any one of the preceding claims, **characterized in that**, for at least one drive unit (5, 6) having a blocking structure:
> the blocking structure of the drive unit (5, 6) also comprises a crank (52, 62) driven to rotate around its axis by said drive motor (51, 61),
> the drive crossmember (55, 65) having a longitudinal axis extending between its two pivoting points (54, 64),
> said axis of rotation of the crank (52, 62) being and remaining parallel to said longitudinal axis of the drive crossmember (55, 65),
> the inter-axis gap, meaning the gap between said axis of rotation of the crank (52, 62) and said longitudinal axis of the drive crossmember (55, 65), being and remaining constant, such that the rotation of the crank (52, 62) causes a rotation of said longitudinal axis of the drive crossmember (55, 65).

4. The drive module according to claim 3, **characterized in that**, for at least two drive units (5, 6) having a blocking structure, for each of these two drive units (5, 6):
> the blocking structure of the drive unit (5, 6) also comprises a crank (52, 62) driven to rotate around its axis by said drive motor (51, 61),
> the drive crossmember (55, 65) having a longitudinal axis extending between its two pivoting points (54, 64),
> said axis of rotation of the crank (52, 62) being and remaining parallel to said longitudinal axis of the drive crossmember (55, 65),
> the inter-axis gap, meaning the gap between said axis of rotation of the crank (52, 62) and said longitudinal axis of the drive crossmember (55, 65), being and remaining constant, such that rotation of the crank (52, 62) causes rotation of said longitudinal axis of the drive crossmember (55, 65).

5. The drive module according to claim 4, **characterized in that** only two drive units (5, 6) per drive device (2) have a blocking structure.

6. The drive module according to claim 5, **characterized in that**, for the third drive unit (4) not having a blocking structure, the structure of said drive unit (4) comprises:
➢ a drive crossmember, driven by the drive motor (41),
➢ a pad holder (3) crossmember, rigidly connected to the pad holder (3) and driven by the drive crossmember,
➢ a single connecting rod (43) respectively connected:
∘ by one of its ends to a pivoting point (44) of the drive crossmember,
∘ by its other end to a pivoting point (44) of the pad holder (3) crossmember.

7. The drive module according to claim 6, **characterized in that**, for said third drive unit (4) not having a blocking structure:
> the structure of said drive unit (4) also comprises a crank (42) driven to rotate around its axis by said drive motor (41),
> the drive crossmember having a longitudinal axis,
> said axis of rotation of the crank (42) being and remaining parallel to said longitudinal axis of the drive crossmember,
> the inter-axis gap, meaning the gap between said axis of rotation of the crank (42) and said longitudinal axis of the drive crossmember, being and remaining constant, such that rotation of the crank (42) causes rotation of said longitudinal axis of the drive crossmember.

8. The drive module according to any one of the preceding claims, **characterized in that**, for one drive unit (4, 5, 6) or for several drive units (4, 5, 6) or for all of the drive units (4, 5, 6), said drive motor or motors (41, 51, 61) are rotary motors driving the rotation of a crank (42, 52, 62) rigidly connected to said drive crossmember (55, 65).

9. The drive module according to any one of the preceding claims, **characterized in that**, for one drive unit (4, 5, 6) or for several drive units (4, 5, 6) or for all of the drive units (4, 5, 6), said drive motor or motors (41, 51, 61) are rotary motors driving the rotation of a worm screw (69) so as to drive said drive crossmember (55, 65) in translation.

10. The drive module according to any one of the preceding claims, **characterized in that**, for each drive device (2):
➢ for each of the three drive units (4, 5, 6) of said drive device (2):
∘ the drive unit (4, 5, 6) drives the pad holder (3):
▪ along a main translational component in a main direction, said main direction being different for each of the three drive units (4, 5, 6),
▪ and along a parasitic translational component in another direction, said other direction being different for each of the three drive units (4, 5, 6),
> each of the three drive units (4, 5, 6) of said drive device (2) incorporates, in its main translational component along its main direction, a compensation for the parasitic translational component of one of the other drive units (4, 5, 6) in this main direction.

11. The drive module according to any one of the preceding claims, **characterized in that** said pivoting points (54, 64) are hinges that can pivot with two degrees of freedom.
